# EUROPEAN PATENT APPLICATION

(11) **EP 2 796 167 A1**
(43) Date of publication of application: **29.10.2014**
(21) Application number: 14000621.4
(22) Date of filing: 21.02.2014
(51) Int. Cl.: A61N 5/06

(54) **LED phototherapy apparatus for skin**

(30) Priority: 22.04.2013 KR 20130044275
(71) Applicant: BS and Co. Ltd, Seoul 143-838 (KR)
(72) Inventor: Min, Sang Ki, Uijeongbu-si, Gyeonggi-do 480-071 (KR); Yoo, Byung Chan, Bucheon-si, Gyeonggi-do 422-750 (KR)
(74) Representative: Müller-Boré & Partner Patentanwälte PartG mbB

(57) **Abstract**

Disclosed therein is an LED phototherapy apparatus for skin including: a support part on which a user lies with the back of the head; a rotation part rotatably hinge-coupled to the support part, the rotation part having a space for accommodating the user's head; a light emitting member formed inside the rotation part; and a control circuit part adapted for supplying electric power to the light emitting member and controlling light intensity and irradiation time. The LED phototherapy apparatus can be utilized as a pillow enabling a user to take sleep on the pillow and irradiate light of wavelength helpful to the skin of the face, the scalp or the neck in the user's sleep so as to provide skin improvement and treatment effects.

## Description

### CROSS-REFERENCE TO RELATED APPLICATION

This application is based on and claims priority from Korean Patent Application No. 10-2013-0044275 filed on April 22, 2013 in the Korean Intellectual Property Office, the disclosure of which is incorporated herein in its entirety by reference.

### BACKGROUND OF THE INVENTION

### Field of the Invention

The present invention relates to an LED phototherapy apparatus for skin, and more particularly, to an LED phototherapy apparatus for skin, which can be utilized as a pillow enabling a user to take sleep on the pillow and irradiate light of wavelength helpful to the skin of the face, the scalp or the neck in the user's sleep.

### Background of the Related Art

Generally, in the modern society, people suffer from various adult diseases due to air pollution, stress, obesity or lack of exercise. Because such diseases are not sufficiently cured just by diagnosis or treatment based on Oriental medicine and Western medical science, health care and therapy methods applying various kinds of alternative medicine are gathering strength.

Color therapy is a method for health care and treatment which receives attention recently and which applies the principle that colored rays having intrinsic wavelengths by colors have different effects on the human body according to the wavelengths.

Effects of therapy methods using light are informed as follows. Red light promotes blood circulation so as to remove the congestion of blood, scarlet light reduces an inflammation so as to activate functions of the brain or kidney, orange light lowers insulin demand quantity, green light stimulates the sympathetic nervous system so as to promote functions of kidney or the liver and bowels and improves detoxification function to pollutants, blue light stabilizes the mental state so as to remove insomnia and relieves asthma, respiratory diseases, muscular dystrophy, digestive problems, ulcer, thyrosis, and so on, purple light controls appetite so as to be helpful to cure of obesity and stabilizes heartbeat, pink light has more effects on mental aspect than physical aspect so as to show vitality to mind and body, yellow light is effective to remedies for colds, and crimson light relieves headache and migraine and gives a sense of psychological stability.

Korean Patent Publication No. 10-2008-0092068 discloses a skin therapy apparatus using light.

The conventional skin therapy apparatus filters rays discharged from an LED through an optical filter and sends the rays of a specific wavelength band to the skin in order to cure and/or improve the skin.

However, such a conventional skin therapy apparatus has a disadvantage in that the skin therapy apparatus is very expensive and it is difficult to secure an installation space because it is complicated in structure and is bulky, and hence, it cannot be utilized for public use.

### SUMMARY OF THE INVENTION

Accordingly, the present invention has been made in view of the above-mentioned problems occurring in the prior art, and it is an object of the present invention to provide an LED phototherapy apparatus for skin, which can be utilized as a pillow enabling a user to take sleep on the pillow and irradiate light of wavelength helpful to the skin of the face, the scalp or the neck in the user's sleep so as to provide skin improvement and treatment effects.

To accomplish the above object, according to the present invention, there is provided an LED phototherapy apparatus for skin including: a support part on which a user lies with the back of the head; a rotation part rotatably hinge-coupled to the support part, the rotation part having a space for accommodating the user's head; a light emitting member formed inside the rotation part; and a control circuit part adapted for supplying electric power to the light emitting member and controlling light intensity and irradiation time.

The support part includes: a base having a flat portion corresponding to the back of the head and side plate portions vertically formed at both sides of the flat portion, the rotation part being hinge-coupled to the side plate portions; and a supporter attached to the upper portion of the flat portion so as to provide a cushiony sense.

The rotation part is formed by combination of an inner cover and an outer cover which are formed in an arc shape, the outer cover has hinge portions which are respectively formed at both end portions of the outer cover and are joined with the side plate portions of the support part, and the light emitting member is joined between the inner cover and the outer cover of the rotation part.

The light emitting member includes a PCB (Printed Circuit Board), an LED (Light Emitting Diode) formed on the front side of the PCB, and a radiator attached to the rear side of the PCB in order to radiate heat.

The LED is a monochromatic LED or an RGB LED emitting multicolored light.

The operation time, color transform time and interval, and color transform sequence of the RGB LED are set by control of the control circuit part.

The control circuit part has a power supply part for supplying electric power, and the power supply part may be a rechargeable battery formed in the supporter or a power cable connected to an external power supply.

The hinge portion includes angle-regulating means for regulating an angle of the rotation part by degrees.

The angle-regulating means includes: a rotary shaft member protrudingly formed on the side plate portion of the support part, the rotary shaft member having a guide plate which has a plurality of through holes formed radially; rotational holders formed at both sides of the rotation part and joined to the rotary shaft member; and ball plungers respectively formed on the rotational holders and selectively joined to the plural through holes of the guide plate.

A finishing cover is joined to the outside of the side plate portion of the support part, and a power source part is joined between the side plate portion and the finishing cover. The power source part includes a power switch which is exposed to the outside through a through hole formed in the finishing cover.

The support part includes a weight body formed therein to add weight.

A non-slip member for preventing slip is formed on the rear cover of the support part.

The control circuit part includes a proximity sensor formed on the inner face of the rotation part, the proximity sensor sensing a distance from the user's target skin which is put on the base member and enabling the user to control light intensity and light emitting time of the LED according to the sensed distance.

The support part includes a sound generation part to reproduce sound or voice.

According to the preferred embodiment of the present invention, the LED phototherapy apparatus for skin can be utilized as a pillow enabling a user to take sleep on the pillow and irradiate light of wavelength helpful to the skin of the face, the scalp or the neck in the user's sleep so as to provide skin improvement and treatment effects.

### BRIEF DESCRIPTION OF THE DRAWINGS

The above and other objects, features and advantages of the present invention will be apparent from the following detailed description of the preferred embodiments of the invention in conjunction with the accompanying drawings, in which:

FIG.1 is a perspective view of an LED phototherapy apparatus for skin according to a preferred embodiment of the present invention;

FIG. 2 is an exploded perspective view of the LED phototherapy apparatus for skin;

FIG. 3 is an enlarged view of a hinge portion of the LED phototherapy apparatus for skin; and

FIGS. 4 to 6 are front views showing a used state of the LED phototherapy apparatus for skin.

### DETAILED DESCRIPTION OF THE PREFERRED EMBODIMENT

Hereinafter, reference will be now made in detail to the preferred embodiment of the present invention with reference to the attached drawings.

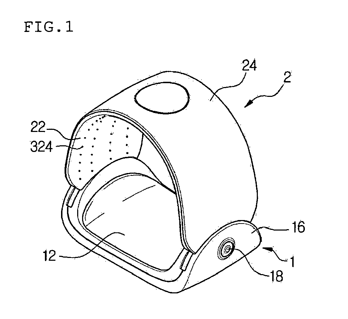

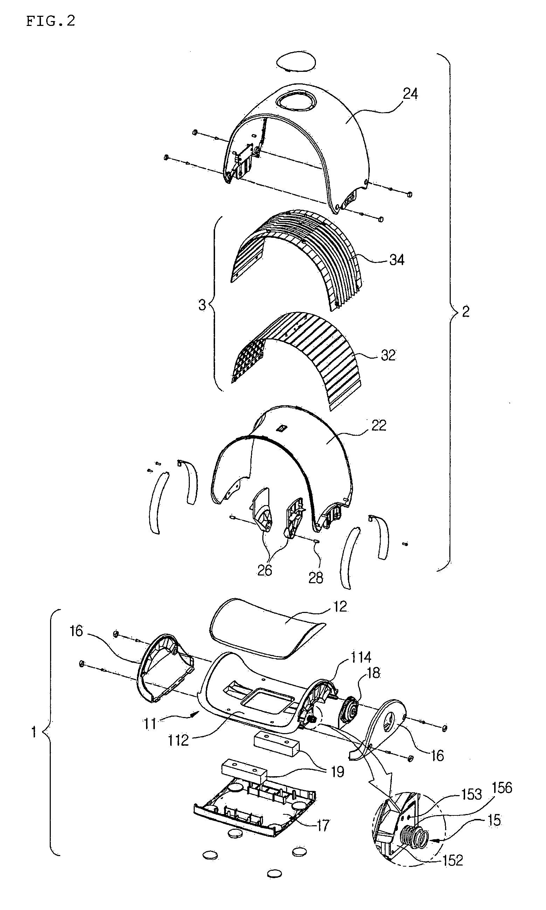

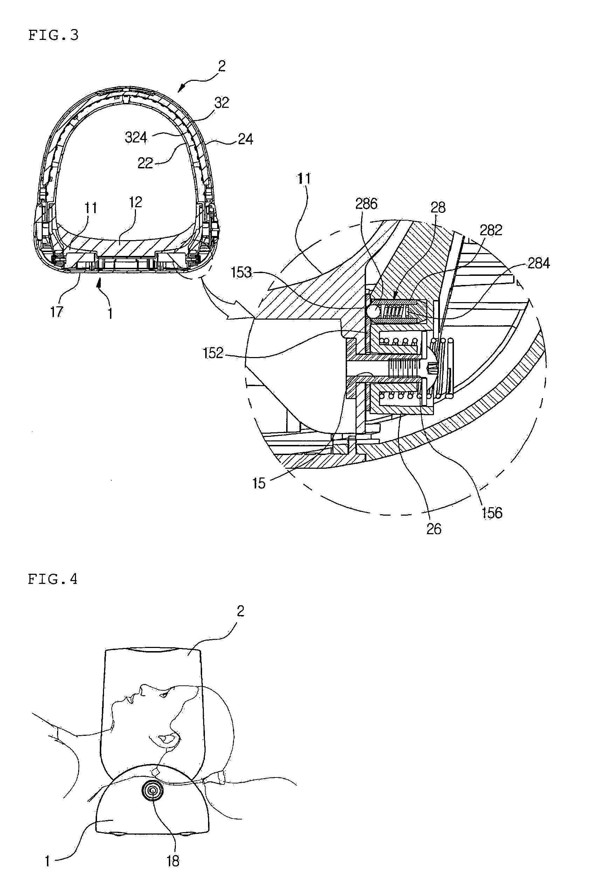

In the attached drawings, FIG.1 is a perspective view of an LED phototherapy apparatus for skin according to a preferred embodiment of the present invention, FIG. 2 is an exploded perspective view of the LED phototherapy apparatus for skin, FIG. 3 is an enlarged view of a hinge portion of the LED phototherapy apparatus for skin, and FIGS. 4 to 6 are front views showing a used state of the LED phototherapy apparatus for skin.

As shown in FIGS. 1 to 6, the LED phototherapy apparatus for skin according to the preferred embodiment of the present invention includes: a support part 1 on which a user lies with the back of the head; a rotation part 2 rotatably hinge-coupled to the support part 1, the rotation part 2 having a space for accommodating the user's head; a light emitting member 3 formed inside the rotation part 2; and a control circuit part adapted for supplying electric power to the light emitting member 3 and controlling light intensity and irradiation time.

In order to be utilized as a pillow, the support part 1 includes: a base 11 which has a flat portion 112 corresponding to the user's back of the head and side plate portions 114 vertically formed at both sides of the flat portion 112, the rotation part 2 being hinge-coupled to the side plate portions 114; and a supporter 12 attached to the upper portion of the flat portion 112 so as to provide a cushiony sense.

The support part 1 has a sound generation part, for instance, an MP3 player, embedded therein so as to play music, and thus, the user can listen to music or study languages.

The rotation part 2 is joined to the upper portion of the support part 1 and is formed by combination of an inner cover 22 and an outer cover 24 which are formed in an arc shape. The outer cover 24 has hinge portions which are respectively formed at both end portions of the outer cover 24 and joined with the side plate portions 114 of the support part 1, and the hinge portions are joined with the support part 1 in a rotatable manner.

The outer cover 24 has rotational holders 26 formed at lower portions of both sides thereof and joined with a rotary shaft member 15 which will be described later.

The light emitting member 3 is joined between the inner cover 22 and the outer cover 24 of the rotation part 2. The inner cover 22 is made of a transparent material to penetrate light or a material to diffuse light.

The material to diffuse light may be made in such a way as to coat PC or ABS powder on glass or transparent plastic material so as to cause diffused reflection.

The light emitting member 3 includes: a PCB (Printed Circuit Board) 32; an LED (Light Emitting Diode) 324 formed on the front side of the PCB 32; and a radiator 34 attached to the rear side of the PCB 32 in order to radiate heat.

It is preferable that the LED 324 be a monochromatic LED or an RGB LED emitting multicolored light.

The operation time, color transform time and interval, and color transform sequence of the RGB LED are set by control of the control circuit part.

The user can manipulate a controller connected to the control circuit part in order to set whether to emit light monochromatically or multicoloredly after setting a wanted operation time.

The control circuit part has a power supply part to supply electric power. The power supply part is embedded in the supporter, and may be a rechargeable battery which is usable for a predetermined period of time in a charged state or a power cable which is directly connected to an external power supply socket so as to supply commercial electricity.

In the meantime, the hinge portion connects the support part 1 and the rotation part 2 so as to rotate the rotation part 2, such that the rotation part 2 can be set to face the user's face, scalp or neck.

Therefore, it is preferable that the hinge portion have angle-regulating means for regulating an angle of the rotation part 2 by degrees.

The angle-regulating means includes: a rotary shaft member 15 protrudingly formed on the side plate portion 114 of the support part 1 and having a guide plate 152 which has a plurality of through holes 153 formed radially; rotational holders 26 formed at both sides of the rotation part 2 and joined to the rotary shaft member 15; and ball plungers respectively formed on the rotational holders 26 and selectively joined to the plural through holes 153 of the guide plate 152.

The rotary shaft member 15 is joined to the rotational holder 26 of the rotation part 2, and a coil spring 156 is joined to the outside of the rotary shaft member 15 such that the rotational holder 26 gets in contact with the guide plate 152. The coil spring 156 pushes one side of the rotational holder 26 so as to provide a strong bearing power, such that the contact state between the rotational holder 26 and the guide plate 152 can be kept.

When the rotational holder 26 and the guide plate 152 are in contact with each other, the ball plungers 28 are joined to the through holes 153 of the guide plates 152 so as to show fixation power.

The ball plunger 28 includes: a spring 284 embedded in a housing 282 joined to the rotational holder 26; and a ball 286 supported by the spring 284 and joined to an opening portion of the housing 282 in such a way as to protrude at a portion. The protruding ball 286 is joined to any one of the plural through holes 153 of the guide plate 152 so as to show fixation power.

The above-mentioned angle-regulating means is just one of examples, and it is obvious that other technical constructions to regulate an angle belong to the technical scope of the present invention.

Meanwhile, a finishing cover 16 is joined to the outside of the side plate portion 114 of the base 11 of the support part 1, and a power source part 18 is joined between the side plate portion 114 and the finishing cover 16.

The power source part 18 includes a power switch which is exposed to the outside through a through hole formed in the finishing cover 16.

In the meantime, a weight body 19 is formed inside the support part 1 to add weight, such that the support part can be fixed without being moved unexpectedly.

Additionally, a non-slip member is formed on a rear cover 17 of the support part 1 so as to prevent slip.

The non-slip member is a thin plate made of rubber or synthetic resin with friction force and has fine protrusions formed on the surface thereof or cohesive components coated on the surface thereof so as to prevent slip.

Meanwhile, the control circuit part further includes a proximity sensor (not shown) formed on the inner face of the rotation part 2.

The proximity sensor senses a distance from the user's skin which is put on the support part 1 and enables the user to control light intensity and light emitting time of the LED 324 according to the sensed distance.

It is preferable that the proximity sensor be an optical sensor, but the present invention is not limited to the above.

Moreover, because the proximity sensor senses the distance, it can prevent that excessive intensity of radiation is irradiated at a close distance.

Meanwhile, because light irradiated from the LED 324 has different wavelengths according to colors and various cells and tissues inside the human body have specific light absorption features by wavelengths, in order to obtain the maximum effect, the user uses light of the wavelength band to be optimally penetrated into the target cells or tissues.

Red light is usable to deep cells or tissues, e.g., sebaceous glands, blue light is useful to intraepidermal therapy, e.g., treatment of actinic keratosis, in PDT (Photo Dynamic Therapy).

In order to have an influence onto as many fibroblasts as possible (it is a goal of some LED therapies), it is preferable to use the deep-penetrating wavelength band.

In case of 660nm, light must penetrate to the 2.3mm depth of the reticular dermis so as to cover the fibroblasts.

Now, the action of LED phototherapy apparatus for skin according to the preferred embodiment of the present invention will be described as follows.

As shown in FIG. 4, when the rotation part 2 is arranged vertically, the LED phototherapy apparatus can radiate light to the user's face.

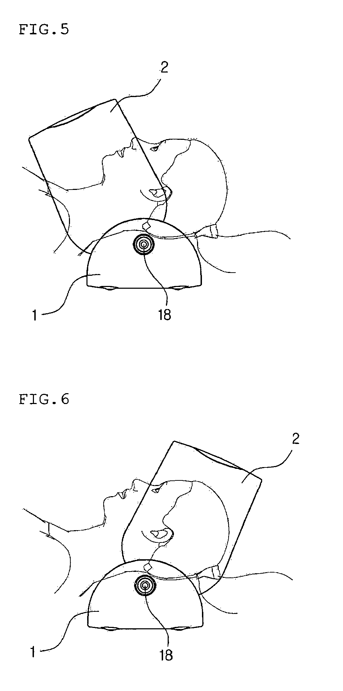

Furthermore, as shown in FIG. 5, when the rotation part 2 is rotated in the clockwise direction, the LED phototherapy apparatus can radiate light to the forehead and the head.

Additionally, as shown in FIG. 6, when the rotation part 2 is rotated in the counterclockwise direction, the LED phototherapy apparatus can radiate light to the neck.

Therefore, the LED phototherapy apparatus according to the preferred embodiment of the present invention can selectively radiate light to a wanted part of the user's body and evenly radiate light of various colors so as to promote skin improvement and treatment.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. An LED phototherapy apparatus for skin comprising:
a support part on which a user lies with the back of the head;
a rotation part rotatably hinge-coupled to the support part, the rotation part having a space for accommodating the user's head;
a light emitting member formed inside the rotation part; and
a control circuit part adapted for supplying electric power to the light emitting member and controlling light intensity and irradiation time.

2. The LED phototherapy apparatus according to claim 1, wherein the support part comprises:
a base having a flat portion corresponding to the user's back of the head and side plate portions vertically formed at both sides of the flat portion, the rotation part being hinge-coupled to the side plate portions; and
a supporter attached to the upper portion of the flat portion so as to provide a cushiony sense.

3. The LED phototherapy apparatus according to claim 1 or 2, wherein the rotation part is formed by combination of an inner cover and an outer cover which are formed in an arc shape, the outer cover has hinge portions which are respectively formed at both end portions of the outer cover and are joined with the side plate portions of the support part, and
wherein the light emitting member is joined between the inner cover and the outer cover of the rotation part.

4. The LED phototherapy apparatus according to any one of claims 1 to 3, wherein the light emitting member comprises a PCB (Printed Circuit Board), an LED (Light Emitting Diode) formed on the front side of the PCB, and a radiator attached to the rear side of the PCB in order to radiate heat.

5. The LED phototherapy apparatus according to any one of claims 1 to 4, wherein the hinge portion comprises angle-regulating means for regulating an angle of the rotation part by degrees.

6. The LED phototherapy apparatus according to claim 5, wherein the angle-regulating means comprises:
a rotary shaft member protrudingly formed on the side plate portion of the support part, the rotary shaft member having a guide plate which has a plurality of through holes formed radially;
rotational holders formed at both sides of the rotation part and joined to the rotary shaft member; and
ball plungers respectively formed on the rotational holders and selectively joined to the plural through holes of the guide plate.

7. The LED phototherapy apparatus according to any one of claims 1 to 6, wherein the support part comprises a weight body formed therein to add weight, and a non-slip member for preventing slip.

8. The LED phototherapy apparatus according to any one of claims 1 to 7, wherein the control circuit part comprises a proximity sensor formed on the inner face of the rotation part, the proximity sensor sensing a distance from the user's target skin which is put on the base member and enabling the user to control light intensity and light emitting time of the LED according to the sensed distance.

9. The LED phototherapy apparatus according to any one of claims 1 to 8, wherein the support part comprises a sound generation part to reproduce sound or voice.
